# EUROPEAN PATENT SPECIFICATION

(11) **EP 4 073 454 B1**
(45) Date of publication and mention of the grant of the patent: **04.11.2026**
(21) Application number: 20878055.1
(22) Date of filing: 09.12.2020
(51) Int. Cl.: F28F 3/14, A61F 7/00

(54) **OPTIMIZED WATER CHANNELS AND FLEXIBLE COOLERS FOR USE IN HEAT EXCHANGE MODULE(S), SYSTEMS, AND METHODS THEREOF**
OPTIMIERTE WASSERKANÄLE UND FLEXIBLE KÜHLER ZUR VERWENDUNG IN (EINEM) WÄRMETAUSCHERMODUL(EN), SYSTEMEN UND VERFAHREN DAVON
CANAUX D'EAU OPTIMISÉS ET REFROIDISSEURS FLEXIBLES À UTILISER DANS UN OU PLUSIEURS MODULES D'ÉCHANGE DE CHALEUR, SYSTÈMES, ET PROCÉDÉS ASSOCIÉS

(30) Priority: 09.12.2019 US 201962974547 P
(43) Date of publication of application: 19.10.2022
(73) Proprietor: Hypothermia Devices, Inc., Inglewood, CA 90302 (US)
(72) Inventor: CUADRA, Daniel, Culver City, CA 90230 (US); COHN, Ryan, Los Angeles, CA 90042 (US); PADULA, Andrew, Laguna Nigel, CA 92677 (US); VERGARA, Julio, L., Los Angeles, CA 90049 (US)
(74) Representative: Marks & Clerk LLP
(86) International application number: PCT/US2020/000047
(87) International publication number: WO 2021/118609

(56) References cited:
- WO-A1-2005/028984
- WO-A1-2017/172836
- KR-A- 20130 068 033
- US-A1- 2005 065 581
- US-A1- 2008 077 211
- US-A1- 2011 172 749

## Description

### FIELD OF THE INVENTION

The invention described herein relates primarily to optimized flexible heat exchange modules (HEMs) that contain a plurality of components including but not limited to, thermoelectric coolers and a series of fluid channels that can be used for heating and cooling. The invention further relates to prognostic, prophylactic, and therapeutic methods useful in cryo- and thermotherapy treatment for various injuries and disorders.

### BACKGROUND OF THE INVENTION

Previously we have described novel methods, systems, modules, and apparatus for use in heating and cooling and a variety of industrial and healthcare applications. See, WO2018/064428 published 05-April-2017; WO2018/064220 published 05-April-2018; WO2017/172836 Published 05-October-2017; WO2017/171719 Published 05-October-2017; US2016/0270952, Published 22-September-2016, US2017/0190102 Published 06-July-2017, and US2018/0098903 Published 12-April-2018. Additionally, we endeavor to further the state of the art in heating and cooling applications as it relates to the treatment of injuries and disorders in humans. As is known in the art, cryo- and thermotherapy treatment of patients is used for a variety of applications, including but not limited to treatment of brain injuries, spinal cord injuries, muscle injuries, joint injuries, avoidance of side effects during chemotherapy treatment, such as hair loss and for neuroprotection after cardiac arrest and neonatal hypoxic ischemic encephalopathy. These treatments are typically afforded by the use of ice packs and/or chemical cool packs that provide incomplete and short-lived cooling, or by pads or caps in which cooling is afforded by circulating chilled fluid, for instances as disclosed in WO 2017/172836 A1.

An aspect of the technology of this disclosure pertains generally to flexible heat exchange modules (HEMs) that contain thermoelectric coolers (TECs) and can be used for heating or cooling.

### SUMMARY OF THE INVENTION

Disclosed herein are three (3) innovations or improvements to a heat exchange module comprising a module or apparatus having a fluid channel and a heat transfer plate in heat transfer relation with fluid in the channel. The module is configured to be operatively position-able with thermally conductive tiles in relation with skin of a patient whereby efficient and effective heat transfer is achieved. The first innovation or improvement relates to an optimized "clamp" style plate in the fluid channel component of the HEM. As disclosed herein, the advantages of the optimized fluid channel(s) will become apparent to one of skill in the art. The second innovation or improvement relates to flexible TECs that can be ergonomically conformed with efficiency and accuracy and can deliver a precise thermal dose to targeted areas on an individual. As disclosed herein, the advantages of the flexible TEC(s) will become apparent to one of skill in the art. The third innovation or improvement relates to specific heating and cooling treatment stations (i.e., for the hand and feet) that provide ergonomically consistent heating and cooling. As disclosed herein, the advantages of the heating and cooling stations will become apparent to one of skill in the art.

Further aspects of the technology described herein will be brought out in the following portions of the specification, wherein the detailed description discloses preferred embodiments of the technology without placing limitations thereon.

### BRIEF DESCRIPTION OF THE FIGURES

**Figure 1****.** Exploded view comparison of the Prior Art Fluid Channel with Two-Sided Embed **(****Figure 1A****)** and the Improved Fluid Channel with Clamp Embed **(****Figure 1B****).**
**Figure 2****.** Exploded view the Improved Fluid Channel with Clamp Embed.
**Figure 3****.** Exploded view of the Prior Art Fluid Channel with Two-Sided Embed.
**Figure 4****.** Cross-Section view comparison of the Prior Art Fluid Channel with Two-Sided Embed **(****Figure 4A****)** and the Improved Fluid Channel with Clamp Embed **(****Figure 4B****).**
**Figure 5****.** Cross-Section view of the Improved Fluid Channel with Clamp Embed.
**Figure 6****.** Cross-Section view of the Prior Art Fluid Channel with Two-Sided Embed.
**Figure 7****.** Exploded view of the Hand Treatment Station.
**Figure 8****.** "Clamp" Style Fluid Channel Thermal Testing.
**Figure 9****.** Simulated HEM Data with Heating Pad.
**Figure 10****.** Simulated HEM Data with No Heating Pad.
**Figure 11****.** Cross-Section view of Flexible Thermo Electric Cooler Embodiment.
**Figure 12****.** Exploded view of Flexible Thermo Electric Cooler Embodiment.
**Figure 13****.** Parameters for Differential Temperature Modelling.
**Figure 14****.** Differential Temperature Model at Time = 0 min. (approx. 10 sec.).
**Figure 15****.** Differential Temperature Model at Time = 2 min.
**Figure 16****.** Differential Temperature Model at Time = 10 min.
**Figure 17****.** Differential Temperature Model at Time = 20 min.
**Figure 18****.** Differential Temperature Model Z-Axis at Time = 2 min.
**Figure 19****.** Differential Temperature Model Z-Axis at Time = 10 min.
**Figure 20****.** Differential Temperature Model Z-Axis at Time = 20 min.
**Figure 21****.** View of Alternative Hand Station Embodiment.
**Figure 22****.** Top View Layout of Various Hand Station Schemes.
**Figure 23****.** Various Configurations of Hand Station Embodiments.
**Figure 24****.** Alternative Design(s) of Hand Station User Interface (UI).
**Figure 25****.** Schematic of Fluid Block Section.
**Figure 26****.** Exploded View of Fluid Block.
**Figure 27****.** Schematic of Fluid Block Assembly
**Figure 28****.** Water Channel Thermal Testing Average Temperature Data.
**Figure 29****.** Water Channel Thermal Testing Data Summary.
**Figure 30****.** Example Peel Test Results.
**Figure 31****.** Examples of Measurable Parameters and Test Results.
**Figure 32****.** Peel Test Pattern for Bond Test.
**Figure** 33. Peel Test Appearance by Embedding Temperature.
**Figure 34****.** Peel Test with Square Plate Design.
**Figure 35****.** Peel Test with Round Plate Design.

### DETAILED DESCRIPTION OF THE INVENTION

### OUTLINE OF SECTIONS

**I.) Overview**
**II.) Novel and Improved Fluid Channel(s)**
**III.) Fluid Block Assembly**
**IV.) Flexible Thermoelectric Coolers ("TECs")**
**V.) Fixed Treatment Stations for Thermo Regulation of Glabrous Skin**
**VI.) Kits** / **Articles of Manufacture**

### I. OVERVIEW

The disclosure includes three innovations or improvements to previous disclosed HEMs which comprise a plurality of TECs and a fluid channel system, specifically designed to transfer heat through direct contact with contoured objects. The first innovation is a "clamp" style fluid channel which possesses several significant advantages over the prior art. The second innovation is a flexible TEC which allows for more targeted and ergonomic heated and cooling. The third innovation is specific heating and cooling stations for specific body parts (e.g., hand and feet). One of ordinary skill in the art will understand and be enabled to design and construct the innovations or improvements of the disclosure of any size, shape, and consistency depending on the desired purpose. In a principal embodiment, HEMs are ergonomic units optimized for heat transfer through the skin for the induction of therapeutic hypothermia and hyperthermia.

### II. NOVEL AND IMPROVED CLAMP STYLE FLUID CHANNEL(S)

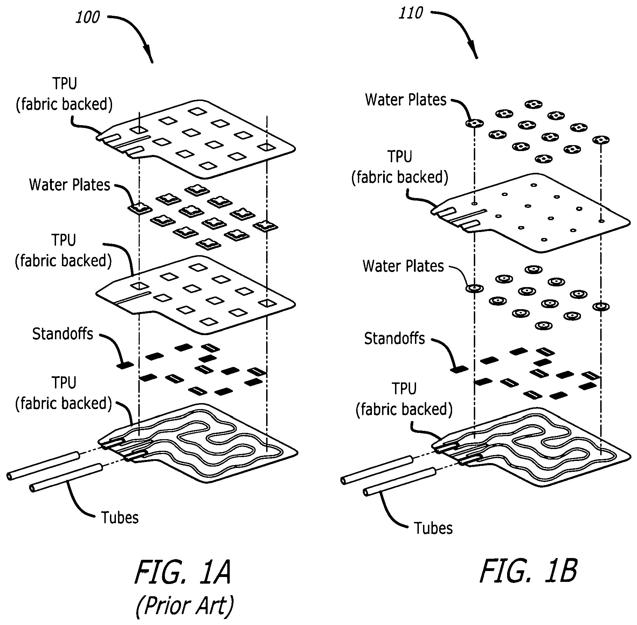

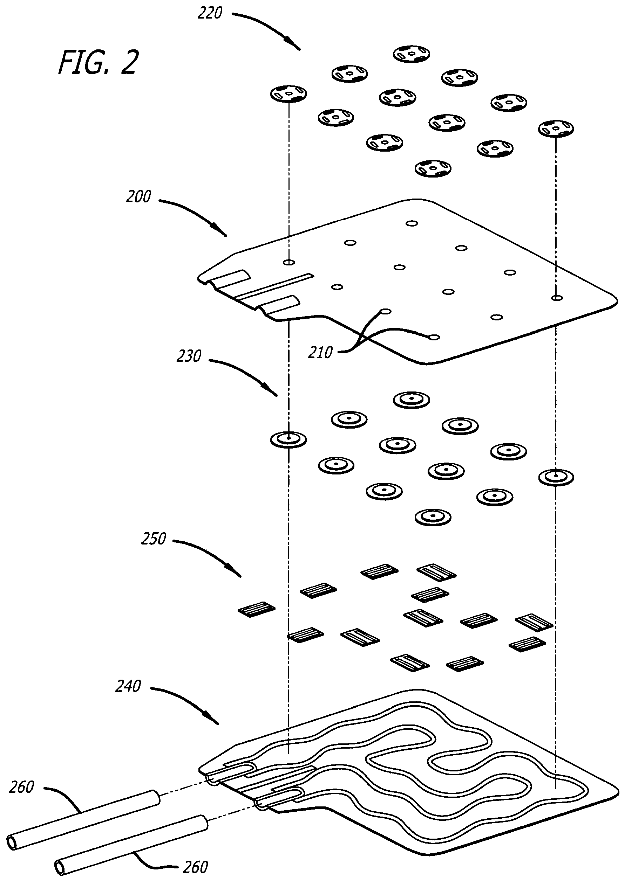

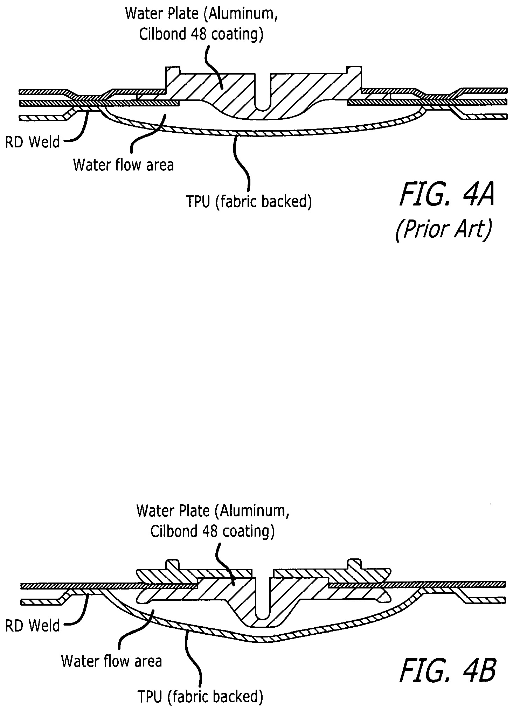

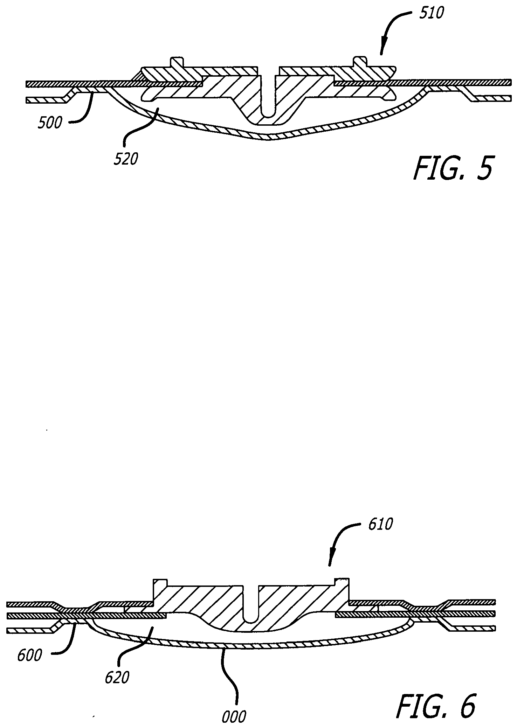

The various components for new and improved "clamp" style fluid channel **(110)** are represented in **Figure 2** and **Figure 5****.** As discussed in this disclosure, the improved "clamp" style fluid channel provides several advantages over the prior art which will be discussed, *infra.* A comparison of the prior art fluid channel and improved "clamp" style fluid channel is show in **Figure 1** and **Figure 4****.**

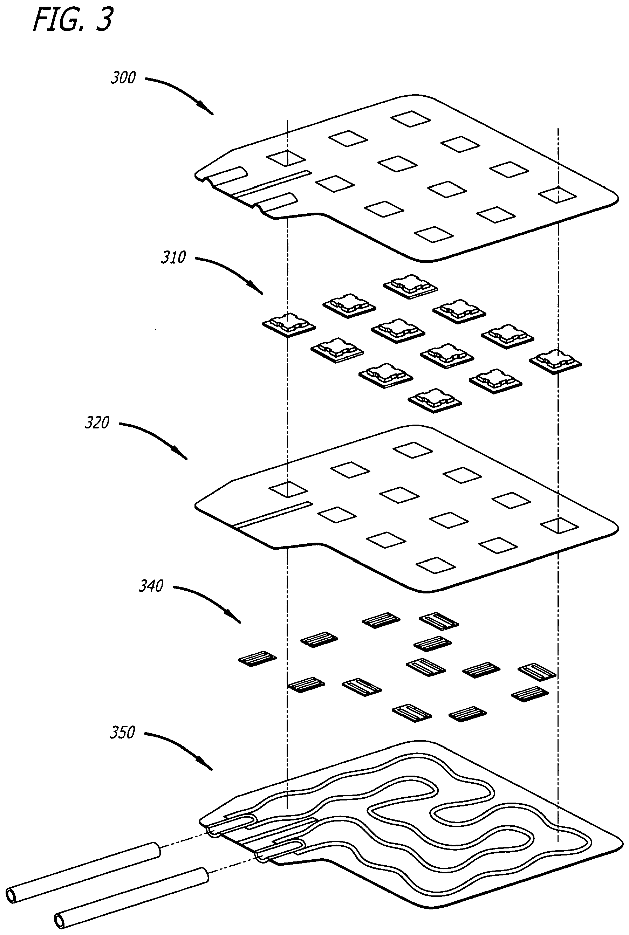

To better understand the advantages of the improved fluid channel, a skilled artisan should view the differences compared with the prior art fluid channel **(100)** which is shown in **Figure 3** and **Figure 6****.** Briefly, the prior art comprises, a first layer **(300)** that can be made from any flexible material, including but not limited to thermoplastic polyurethane sheets ("TPU"). The first layer of material has cut outs **(330)** directly under the plate to allow the plate to be in direct contact with the fluid, thus increasing heat transfer. Plates **(310)** which directly contact fluid flowing in the channel are embedded between two layers of materials. Generally speaking, the plates can be made of any thermally conductive material, including but not limited to aluminum, and may or may not include an adhesion primer coating. A second layer **(320),** which similar to the first layer, may be any flexible material, including but not limited to fabric backed TPU. Additionally, standoffs **(340)** may be attached via RF weld **600** or otherwise, to the material on the side opposite to the plates' elevated platform **610** to maintain fluid flow and prevent channel collapse when the fluid channel assembly is flexed. A third sheet of material **(350)** is attached, via RF weld **600** or otherwise, onto the assembly to create a continuous fluid path **620.** Finally, inlet and outlet tubes **(360)** made from the same material, which similar to the first, second, and third layers may include, but are not limited to TPU, are joined, by RF weld **600** or other process, into the assembly to connect to an external interface.

In a typical embodiment, the circulating fluid can be water, distilled water, or distilled water with an antimicrobial agent to prevent the long-term growth of microbes which may interfere with the operation of the system. In other embodiments, additional additives can be included in the fluid, such as (among others) agents to reduce the surface tension of water, agents to protect the life of internal components, agents to buffer against pH changes, and coloring agents for the visualization of long-term chemical changes. In yet other embodiments, the system can take advantage of synthetic fluids with improved heat conductivity with respect to that of water.

The various components of the improved "clamp" style fluid channel **(110)** comprise the following elements which differ significantly in form compared to the prior art **(110)** and result in significantly better quality and stability. Briefly, a first layer **(200)** made from any flexible material, including but not limited to thermoplastic polyurethane sheets ("TPU"). The first layer of material has cut outs **(210)** in a shape that can comprise a uniform grid or can be modified to any shape necessary to achieve uniform heat transfer properties and to conform to the surface that is being treated. A first Plate **(220)** and a second Plate **(230)** which are "clamped" together at the point of the cut out (210) by mechanical fasteners (e.g. bolt or integral male/female threads on the upper and lower clamp). The second Plates **(230)** directly contact fluid flowing in the channel and are embedded between the first layer **(200)** and a second layer of material **(240),** which similar to the first layer **(200)** are made from any flexible material, including but not limited to thermoplastic polyurethane sheets ("TPU").

The fluid channel assembly may include a thermally conductive compressible material or thermally conductive paste at the interface between the first and second plate to ensure proper surface contact for heat transfer. Additionally, standoffs **(250)** may be attached via RF weld **500** or otherwise, to the material on the side opposite to the plates' elevated platform **510** to maintain fluid flow and prevent channel collapse when the fluid channel assembly is flexed. The second sheet of material **(240)** is attached, via RF weld **500** or otherwise, onto the assembly to create a continuous fluid path **(520).** Finally, inlet and outlet tubes **(260)** made from the same material, which similar to the first, second, layers may include, but are not limited to TPU, are joined, by RF weld **500** or other process, into the assembly to connect to an external interface.

In a typical embodiment, the circulating fluid can be water, distilled water, or distilled water with an antimicrobial agent to prevent the long-term growth of microbes which may interfere with the operation of the system. In other embodiments, additional additives can be included in the fluid, such as (among others) agents to reduce the surface tension of water, agents to protect the life of internal components, agents to buffer against pH changes, and coloring agents for the visualization of long-term chemical changes. In yet other embodiments, the system can take advantage of synthetic fluids with improved heat conductivity with respect to that of water.

It will be apparent to one of skill in the art that the novel and improved "clamp" style provides several advantages over the prior art. First, the prior art two-sided embed does not provide a secure seal compare to the improved "clamp" style. This will be apparent since the prior art provided two layers on the top and the bottom of the Plate(s). The improved "clamp" embed provides a "clamp" of a first and second Plate surrounding the first layer of material, thereby creating a significantly tighter seal. This non-obvious property of a more secure seal became known after significant failures of the prior art two-sided embed during production. Notably, the prior art two-sided embed possessed a failure rate of approximately thirty (30%) percent during manufacturing, which represents significant costs in wasted production runs. This is due, in part, to the fact that the tooling used to manufacture the prior art two-sided embed consisted of a hot tooling apparatus which came in contact with the layered TPU during the sealing process. The direct contact caused damage to the exposed edges of the TPU layer which allows fluid to ingress through the cross section of the material. Because of this, the manufacturing process was very time consuming and had a significant failure rate.

Conversely, the novel and improved "clamp" style embed offers several advantages. First, in addition to the tighter seal due to the "clamp" embodiment, the production can be done much faster than that the prior art two-sided embed. Second, the plate geometry covers the exposed edges of TPU material, and the hot tooling only comes in contact with the plates, minimizing the material degradation and preventing water ingress. Third, because of this, the failure rate during manufacturing is significantly less. Finally, since the prior art two-sided embed requires post-processing step(s) to prevent fluid leakage due to the material degradation and water ingress, the overall cost of the "clamp" embed production is reduced because the improved embodiment does not experience this issue and therefore post-processing step(s) are not required.

In one embodiment, the invention comprises, an improved "clamp" style fluid channel apparatus comprising, (i) a first layer, (ii) a first water plate, (iii) a second water plate, and (iv) a second layer, whereby the first water plate and second water plate are "clamped" to create a seal against the first layer.

In a further embodiment, the invention comprises, an improved "clamp" style fluid channel apparatus comprising, (i) a first layer, (ii) a first water plate, (iii) a second water plate, and (iv) a second layer, as substantially shown in **Figure** 2, whereby the first water plate and second water plate are "clamped" to create a seal against the first layer as substantially shown in **Figure 5****.**

In one embodiment, the invention comprises, an improved "clamp" style fluid channel apparatus comprising, (i) a first layer, (ii) a first water plate, (iii) a second water plate, and (iv) a second layer, whereby the first water plate and second water plate are "clamped" to create a seal against the first layer, further comprising a standoff, whereby the standoff is attached to the material on the side opposite to the plates' elevated platform to maintain fluid flow and prevent channel collapse when the fluid channel assembly is flexed.

In one embodiment, the invention comprises, an improved "clamp" style fluid channel apparatus comprising, (i) a first layer, (ii) a first water plate, (iii) a second water plate, and (iv) a second layer, whereby the first water plate and second water plate are "clamped" to create a seal against the first layer, further comprising a standoff, whereby the standoff is attached to the material on the side opposite to the plates' elevated platform to maintain fluid flow and prevent channel collapse when the fluid channel assembly is flexed, further comprising inlet and outlet tubes connect into the assembly to connect to an external interface.

In another aspect of the disclosure, the invention comprises a method of manufacturing the improved "clamp" style fluid channel embed substantially in the form of **Figure 2****.**

In another embodiment, the invention comprises a "clamp" style fluid channel embed by a process comprising:
(i) The first layer is placed in between the upper and lower metal plates;
(ii) External heat and opposing pressure are applied to each plate, resulting in localized melting of the layer;
(iii) The melted material of the layer forms a bond with both plates on either side, sealing the clamp joint.

In another embodiment, the invention comprises a "clamp" style fluid channel embed by a process comprising:
(i) The first layer is placed in between the upper and lower metal plates coated with an adhesion promoter;
(ii) External heat and opposing pressure are applied to each plate, resulting in localized melting of the layer;
(iii) The melted material of the layer forms a bond with both plates on either side, which is enhanced by the adhesion promoter, sealing the clamp joint.

One of ordinary skill in the art will appreciate and be enabled to make variations and modifications to the disclosed embodiment without altering the function and purpose of the invention disclosed herein. Such variations and modifications are intended within the scope of the present disclosure.

### III. FLUID BLOCK ASSEMBLY

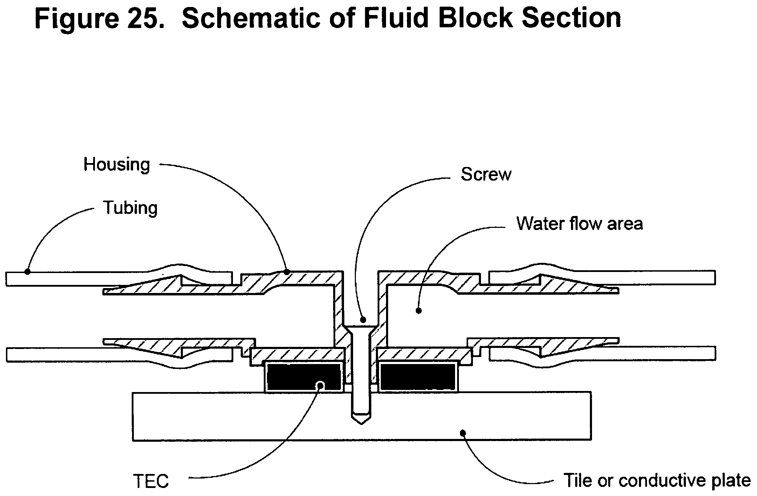

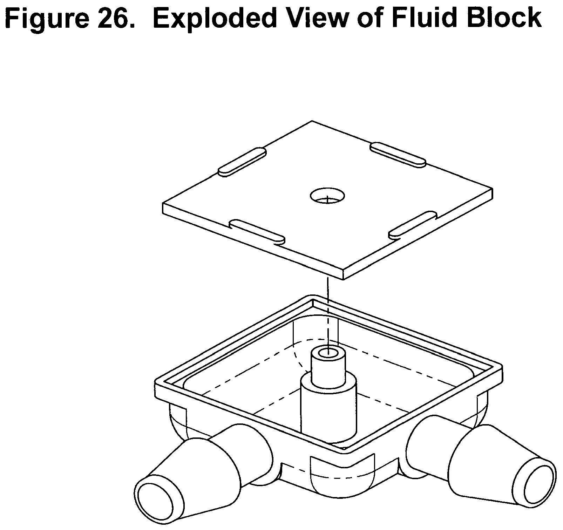

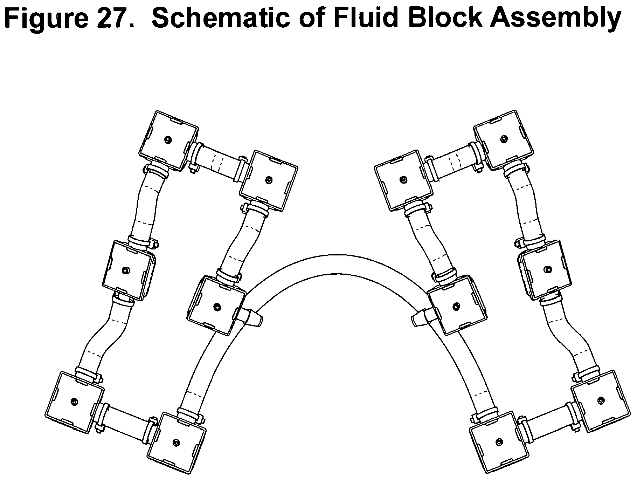

In another embodiment, the disclosure teaches a new and improved fluid channel assembly which is exemplified in **Figure 25****,** **Figure 26****,** and **Figure 27****.** One of ordinary skill in the art will appreciate that the improved embodiment(s) provide a modular solution for fluid channel production and prototyping. In this method of making a fluid channel a thermally conductive metal platform is adhesive bonded to a rigid plastic frame, creating an enclosure which allows fluid, such as water, to pass through. See, **Figure 25** and **Figure 26****.** Each fluid block features a clearance hole which allows the block to be fastened through a TEC and into a threaded hole in any thermally conductive material which forms the tile or patient contact surface. It should be noted that no mounting holes are required in the patient-facing side of the contact surface, improving cosmetic appearance, and making the surface easier to clean and maintain. Accordingly, a series of rigid blocks can be joined by flexible tubing connected to the fluid blocks by integral barb fittings. See, **Figure 27****.** This allows an infinite number of possible position configurations for the fluid channel. One of skill in the art will appreciate several advantages over the currently state of the art. First, a lack of tooling required to assemble a unique configuration. Second, the improved design allows for more efficient design of co-planar configurations (i.e., for mounting to a 3D topography). Third, greater aesthetic appearance since there are no mounting holes visible to the patient / end user. Fourth, the improved fluid channel design allows for easy clear and repair thereby increasing useful life and product integrity.

### IV. FLEXIBLE THERMOELECTRIC COOLERS ("TECs")

A second innovation of the disclosure relates to improved thermoelectric coolers ("TECs") that are flexible and more readily conform to the contact surface while minimizing heat loss. Based on a brief review of our previous endeavors (See, WO2018/064428), we have shown that our heat exchange modules (HEMs) comprise TECs that are used for heating and cooling in various applications. Generally speaking, and as to what has been previously taught, individual TECs, or a plurality of TECs organized in arrays, act as direct-contact heat pumping elements. In a standard embodiment, the outer surfaces of the TECs exchange heat through fluid channels (See, Novel and Improved Clamp Style Fluid Channels, *supra).* Furthermore, a HEM is based around an array of TECs which transmit heat to or from the user at the skin level. The TECs are wired in various arrays and provide uniform control of temperature over the area of the HEM. Each TEC is paired with a temperature sensor that provides feedback by measuring the temperature of the thermally conductive surface in contact with the user, known as a tile.

Tiles are constrained in a geometric pattern appropriate to the anatomy for which the HEM is intended by attachment to a flexible frame. The flexible frame can be made of any flexible material, including but not limited to thermoplastic polyurethane sheets (TPU). The frame retains the tiles and provides a continuous surface barrier between the user and the TECs and other internals of the HEM.

A watertight bladder known as a fluid channel, *supra,* connects to the TEC array and provides a method of heat extraction from the system. Thermally conductive plates are embedded into a TPU bladder in a pattern mirroring the geometry of the tiles. Each TEC is mounted to a plate that transfers heat from the TEC into a circulating body of fluid. Fluid carries the heat away from the TECs and releases it through a radiator in an externally connected console.

In one embodiment, the subassembly of TECs, tiles, and fluid channel can be packaged for use inside a soft good that provides a biocompatible material comfort layer between the user and the tiles, hook-and-loop straps, and/or elements necessary for affixing the device to the user's body, and an air bladder to adjust the pressure and fit.

Based on the foregoing, one of ordinary skill in the art will understand and be enabled to design and construct TECs of the disclosure of any size, shape, and consistency depending on the desired purpose.

In view of the above, researchers have shown that part of the energy is wasted by traditional TEC design because of an imperfect contact with bodily tissues due to their rigidity. Additionally, the application of personal thermoregulation devices is gaining popularity. However, the development of active heating and cooling garments is far more challenging and largely unexplored since most heating and cooling devices are bulky and difficult to integrate into a garment or other soft good. In addition, previous attempts to develop improve TECs have not exhibited sustained active cooling performance without the aid of a water heat sink. See, HONG, et. al., Sci. Adv. 2019;5.

As noted above, a HEM of the disclosure generally comprises an array of TECs. In prior embodiments, the TECs are made of rigid, non-flexible material.

Accordingly, there is a need in the art for flexible TECs that provide targeted focused heating and cooling to a user while being able to maintain sustained heating and cooling. In one aspect of the current disclosure, a hybrid approach is found to be new and useful. The approach utilized fluid-based fluid channels as well as solid-state flexible TECs. The flexible TECs are placed on the non-water plate side of a HEM. The result provides a precise thermal dose to targeted areas on an individual while at the same time maintaining consistent, long-term heating and cooling to the user.

In one embodiment, the flexible TEC comprises a solid-state thermoelectric cooling technology. Briefly, the thermoelectric effect refers to phenomena by which either a temperature difference creates an electric potential, or an electric potential creates a temperature difference. These phenomena are known more specifically as the Seebeck effect (creating a voltage from temperature difference), Peltier effect (driving heat flow with an electric current), and Thomson effect (reversible heating or cooling within a conductor when there is both an electric current and a temperature gradient). Generally speaking, all materials have a nonzero thermoelectric effect, in most materials it is too small to be useful. However, low-cost materials that have a sufficiently strong thermoelectric effect (and other required properties) are also considered for applications including power generation and refrigeration. The most commonly used thermoelectric material is based on bismuth telluride (Bi₂Te₃). It should be noted that any material can be used as long as the material possesses (i) high electrical conductivity, (ii) low thermal conductivity, and (iii) high Seebeck coefficient.

Additionally, an elastomer is a polymer with the property of "elasticity," generally having notably low Young's modulus and high yield strain compared with other materials. The term is often used interchangeably with the term "rubber". Elastomers are amorphous polymers existing above their glass transition temperature, so that considerable segmental motion of the polymer chain is possible and therefore; it is expected that they would also be very permeable. Examples of elastomers include natural rubbers, styrene-butadiene block copolymers, polyisoprene, polybutadiene, ethylene propylene rubber, ethylene propylene diene rubber, silicone elastomers, fluoroelastomers, polyurethane elastomers, and nitrile rubbers.

In addition, A copolymer is a polymer derived from more than one species of monomer. The polymerization of monomers into copolymers is called copolymerization. Copolymerization is used to modify the properties of manufactured plastics to meet specific needs, for example to reduce crystallinity, modify glass transition temperature, control wetting properties or to improve solubility. Commercial copolymers include acrylonitrile butadiene styrene (ABS), styrene/butadiene co-polymer (SBR), nitrile rubber, styrene-acrylonitrile, styrene-isoprene-styrene (SIS) and ethylene-vinyl acetate, all formed by chain-growth polymerization.

Accordingly, there is a need for thermoelectric materials to be integrated with a flexible material to create a flexible TEC.

In one embodiment, the invention comprises a flexible TEC comprising a thermoelectric material selected from the group consisting of Bi₂Te₃, Bi₂Se₃, PbTe (thallium-doped lead telluride alloy), Ba₈Ga₁₆Ge₃₀, Ba₈Ga₁₆Si₃₀, Mg₂B^{IV} (B^{IV} = Si, Ge, Sn), ZnO, MnO₂, NbO₂, NbFeSb, NbCoSn, and VFeSb.

In one embodiment, the invention comprises a flexible TEC comprising an elastomer.

In one embodiment, the invention comprises a flexible TEC comprising a copolymer.

Methods of making flexible TECs are known in the art. See, for example, HONG, et. al., Sci. Adv. 2019;5 and KISHORE, et. al., Nature Communications 10:1765 (2019).

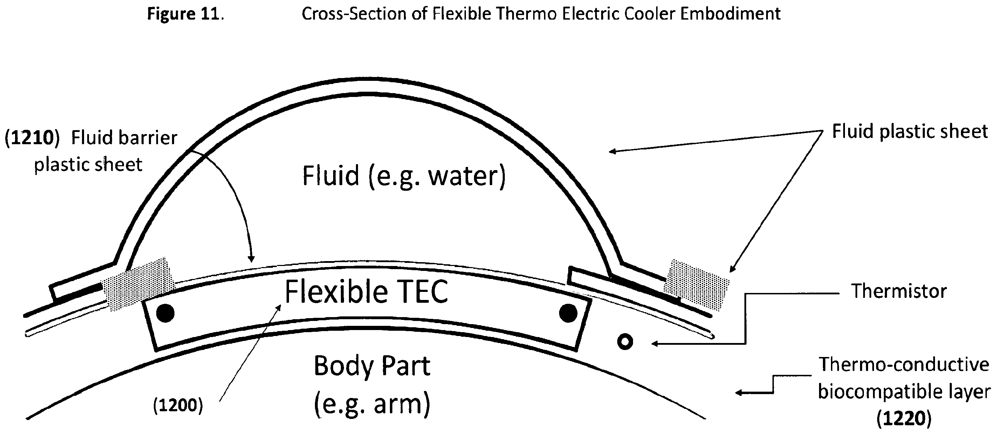

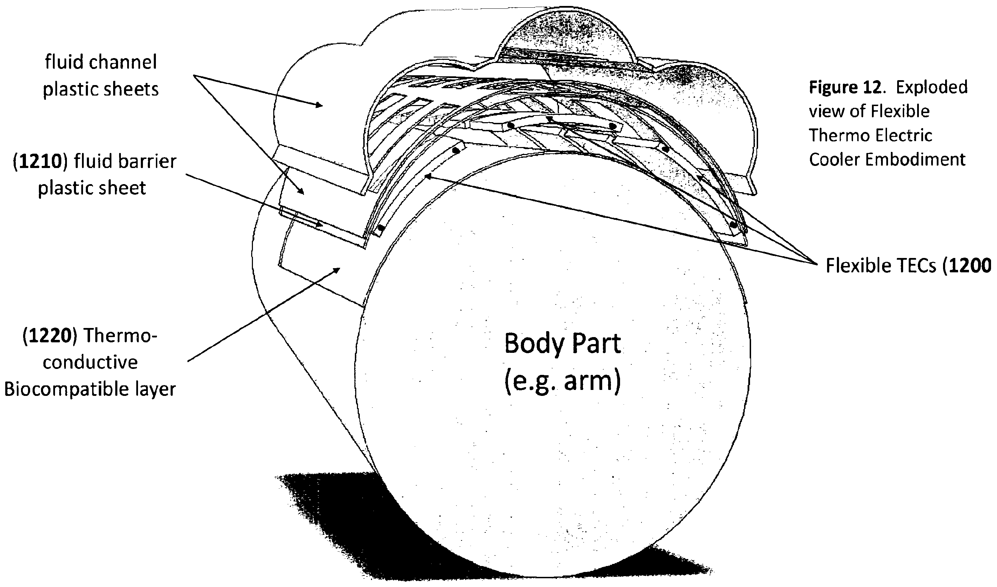

Accordingly, in one embodiment, a HEM as previous disclosed (WO2018/064428) comprises a flexible TEC of the invention. In a further embodiment, a HEM as previous disclosed comprises a flexible TEC as shown in **Figure 11** and **Figure 12****.** Briefly, a flexible TEC of the invention **(1200)** is located between a body part (e.g., an arm) and a fluid barrier plastic sheet (e.g., TPU, etc.) **(1210).** The flexible TEC may be indirect contact with the skin or may be in contact with a thermo-conductive biocompatible layer **(1220).** The result provides optimized targeted heating and cooling to a user while being able to maintain sustained heating and cooling on the target area. An additional advantage of the use of flexible TECs in this embodiment is that they can be ergonomically put in direct contact (or through a thin thermo-conductive interface layer) with body parts that exhibit a curvature difficult to overcome with a rigid plate, which thereby increases efficacy of treatment. This close physical contact undoubtedly permits the optimization of the heat exchange process necessary for cooling/heating of body parts and allows more uniform skin contact, fewer pressure points and a higher degree of patient comfort. **Figure(s) 11 and 12.**

### V. FIXED TREATMENT STATIONS FOR THERMO-REGULATION OF GLABROUS SKIN

A third innovation of the disclosure relates to fixed frame therapeutic station(s) (e.g., for the hand(s), feet, etc.) that are used to improve the controlled radiator function of the glabrous skin in humans. Studies have shown that heat loss through the glabrous skin is more variable and can reach higher values than through non-glabrous skin. Moreover, vacuum-enhanced heat extraction from the glabrous skin reduces the rate of core temperature rise during heat exposure and exercise and thus improves performance. See, HELLER, et. al., Disruptive Sci. and Tech., vol. 1, no. 1 (2012). *See also,* US Patent No. 7,122,047. Thus, it will become apparent to one of ordinary skill that targeted thermoregulation of the glabrous skin in humans can be beneficial on a number of levels. First, it will greatly assist in the design of thermally protective gear, such as softgoods for athletic and military use. Second, the ability to effectively manage and thermo-regulate glabrous skin may also inhibit fatigue in sports / competition and allow for more effective recovery during physical therapy. Studies have shown that the effects of cooling (or heating) multiple glabrous skin areas are additive. *See,* GRAHN, et. al. J. Biomech. Eng., 131:071005 (2009). Third, utilizing the additive effects of thermo-regulating glabrous skin may also influence medical conditions that are affected by temperature change. For example, cooling to chemotherapy or radiation therapy in cancer patients. Maintaining steady state temperature perioperatively, peripheral neuropathy, etc. In fact, studies have shown inserting heat into the core of hypothermic patients recovering from the effects of anesthesia have shown some benefit. See, GRAHN, et. al., J. Appl. Physio., 85:pp. 1643-1648 (1998).

The prior art teaches several types of embodiments that purportedly teach the use of heating and cooling glabrous skin surfaces with vacuum-enhanced systems. *See for example,* U.S. 7,122,047; U.S. 7,947,068; U.S. 2016/0374853; and U.S. 2007/0060987. However, these systems are disadvantageous compared to the embodiments in the current disclosure for the following reasons. First, the prior art systems require constant monitoring of vasoconstriction and/or vasodilation conditions. Second, the systems are bulky and not mobile due to the fact that they possess vacuum enhanced systems. Third, there is not ability to provide a differential temperature to various areas of the body.

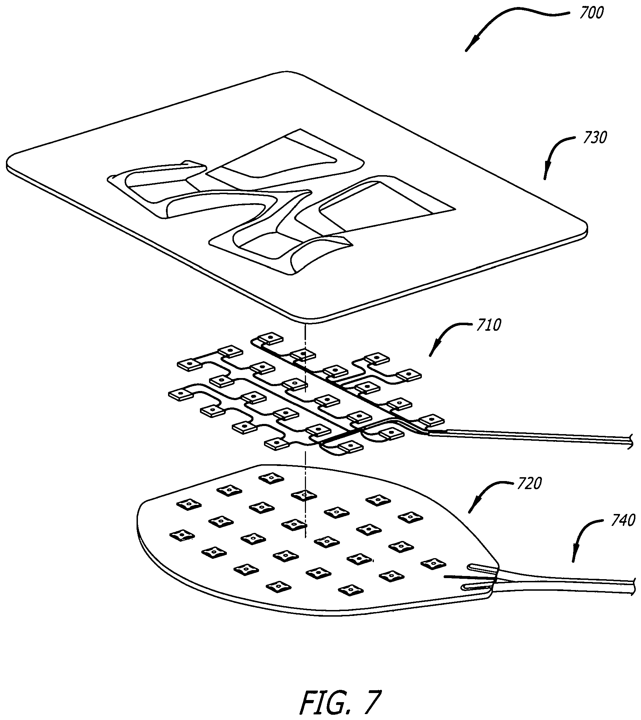

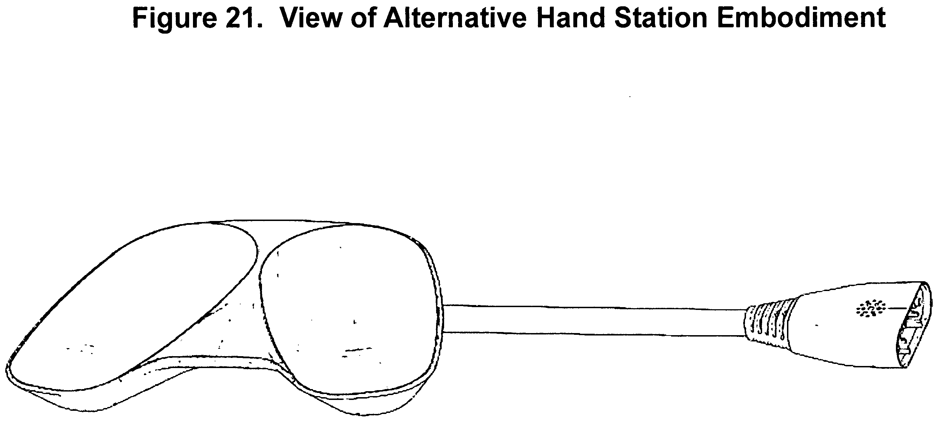

In contrast, the current disclosure provides fixed frame therapeutic stations to be used for heating and cooling therapies. The embodiments disclosed herein builds upon the previous HEM system(s) (See, Hypothermia Devices, Inc., Los Angeles, CA) and are further described in **Figure 7** and **Figure 21****.** As is show, **Figure 7** is an exploded view of a fixed frame hand station of the disclosure **(700).** In reference thereto, the TEC array is captured between the fixed frame thermal interface layer **(710)** and the fluid channel subassembly **(720).** As has been shown, a compressible thermally conductive material or a thermally conductive paste can be used to ensure thermal contact between the TEC array and both the fluid channel subassembly and the fixed frame thermal interface layer of the hand station **(730).** The fixed frame can be made from any thermally conductive material, but a preferred embodiment is aluminum. Finally, inlet and outlet tubes **(740),** are joined, by RF weld or other process, into the assembly to connect to an external interface. It will be apparent to one of skill in the art that the fixed frame can be molded to any suitable body part comprising a glabrous skin surface (e.g., the hands and feet).

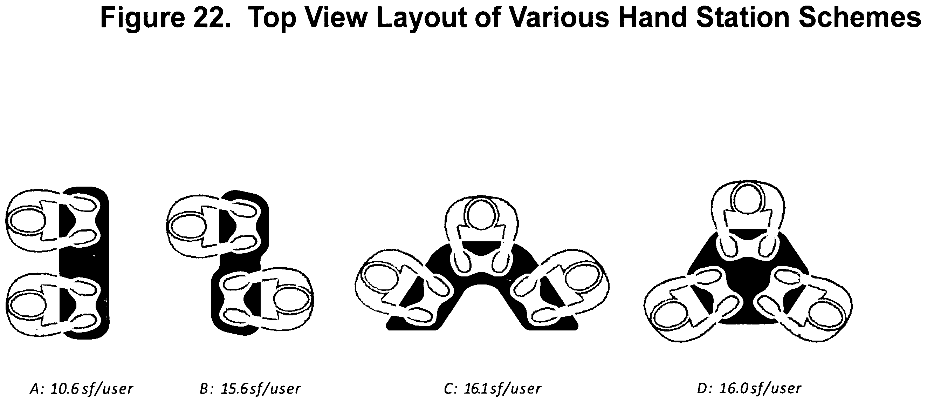

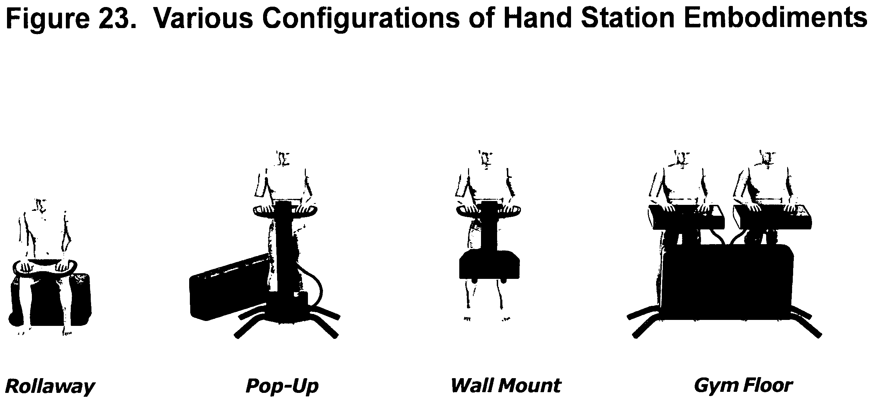

The hand station of the disclosure can be arranged so as to maximize the spacing and efficiency for the end user. For example, as shown in **Figure 22****,** the hand station(s) can be arranged in a plurality of formats depending on the available space, the number of end users, and the activity. These "hubs" can be installed in gyms, or can be built portably for use on sports sidelines, or at events. Each hub concept shown is evaluated based on the number of square feet it occupies per user (sf/user). In addition, as shown in **Figure 23****,** each hand station of the disclosure can be configured for specific type of product modality depending on the needs of the user. For example, non-limiting examples of product configurations are rollaway (self-contained system), pop-up, wall mounted, or stationary placement (for example, on a gym floor).

In one embodiment, a hand station of the disclosure can be integrated with a plurality of sanitation modalities. This allows users to clean the unit before and after each use. One of ordinary skill in the art will understand and appreciate that sanitation modalities can be automatic or manual and may be portable or permanently fixed to the hand station.

In a further embodiment, a hand station of the disclosure can be integrated with a plurality of sensors and metrics to monitor and analyze various aspects of a user's performance. For example, treatment time can be tracked, optionally notifying a user when a recommended recovery period has elapsed. Of note, a capacitive sensor can be used to detect when a user has started treatment. In addition, a heart rate (pulse) metric can be employed. A pulse can be measured by detecting electrical pulses measured by two (2) electrodes attached to the user (preferably beneath the hands or wrists). Alternatively, a LED and photosensitive diode can detect pulse. In addition, an electrocardiogram (EKG/ECK), blood oxygen saturation (SpO₂), and body mass index (BMI) can also be recorded using methods known in the art.

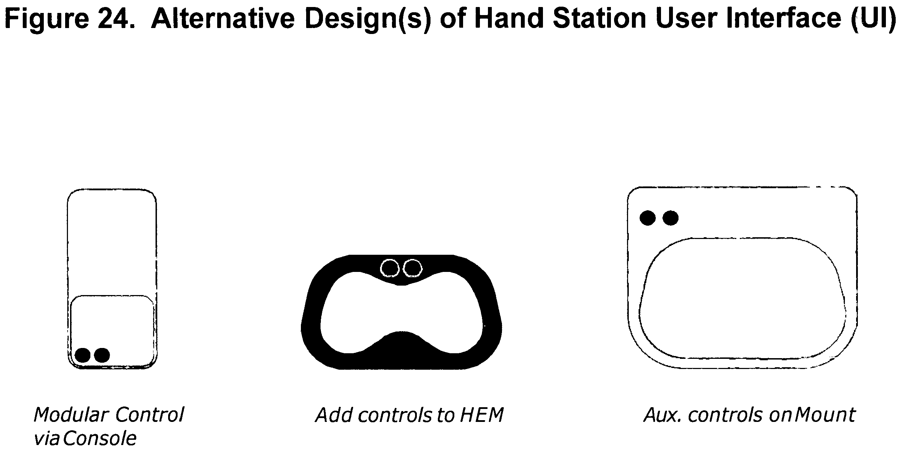

In a further embodiment, a plurality of user interface (UI) designs can be employed. For example, a UI can be integrated via a modular console, a mounting plate, or a HEM console. Non-limiting exemplary Ul's are show in **Figure 24****.**

In one embodiment, the invention comprises, a fixed frame therapy station apparatus comprising, (i) a fixed frame station, (ii) a fluid channel subassembly, and (iii) a controller.

In one embodiment, the invention comprises, a fixed frame therapy station apparatus comprising, (i) a fixed frame station, wherein the fixed frame is molded in the shape of a human hand, (ii) a fluid channel subassembly, wherein the fluid channel subassembly comprises a "clamp" style fluid channel of the disclosure, and (iii) a controller.

In one embodiment, the invention comprises, a fixed frame therapy station apparatus comprising, (i) a fixed frame station, wherein the fixed frame is molded in the shape of a human foot, (ii) a fluid channel subassembly, wherein the fluid channel subassembly comprises a "clamp" style fluid channel of the disclosure, and (iii) a controller.

In one embodiment, the invention comprises, a fixed frame therapy station apparatus comprising, (i) a fixed frame station, (ii) a fluid channel subassembly, and (iii) a controller as substantially shown in **Figure 7****.**

In one embodiment, the invention comprises, a fixed frame therapy station apparatus comprising, (i) a fixed frame station, (ii) a fluid channel subassembly, and (iii) a controller as substantially shown in **Figure 7** and wherein the fluid channel comprises a "clamp" style fluid channel as shown substantially in **Figure 5****.**

In one embodiment, the invention comprises, a fixed frame therapy station apparatus comprising, (i) a fixed frame station, wherein the fixed frame is molded in the shape of a human hand, (ii) a fluid channel subassembly, wherein the fluid channel subassembly comprises a "clamp" style fluid channel of the disclosure, and (iii) a controller substantially shown in **Figure 7** and wherein the fluid channel comprises a "clamp" style fluid channel as shown substantially in **Figure 5****.**

In one embodiment, the invention comprises, a fixed frame therapy station apparatus comprising, (i) a fixed frame station, wherein the fixed frame is molded in the shape of a human foot, (ii) a fluid channel subassembly, wherein the fluid channel subassembly comprises a "clamp" style fluid channel of the disclosure, and (iii) a controller substantially shown in **Figure 7** and wherein the fluid channel comprises a "clamp" style fluid channel as shown substantially in **Figure 5****.**

In one embodiment, the invention comprises, a fixed frame therapy station apparatus comprising, (i) a fixed frame station with a plurality of contact areas, (ii) a fluid channel subassembly, and (iii) a controller as substantially shown in **Figure 21****.**

In one embodiment, the invention comprises, a fixed frame therapy station apparatus comprising, (i) a fixed frame station with a plurality of contact areas, (ii) a fluid channel subassembly, and (iii) a controller as substantially shown in **Figure 21** and wherein the fluid channel comprises a "clamp" style fluid channel as shown substantially in **Figure 5****.**

One of ordinary skill in the art will appreciate and be enabled to make variations and modifications to the disclosed embodiment without altering the function and purpose of the invention disclosed herein. Such variations and modifications are intended within the scope of the present disclosure defined by the appended claims.

### EXAMPLES

Various aspects of the invention are further described and illustrated by way of the several examples that follow, none of which is intended to limit the scope of the invention.

### Example 1: "Clamp" Style Fluid Channel Thermal Testing.

Thermal testing of the "clamp" style fluid channel was performed to determine whether the "clamp" style modality could perform better than the previous embodiment. Many variations of the "clamp" style modality were tested, including plates with varying area of contact between the plates and using thermally conductive paste between the two plates. By way of background, previous testing showed that the plate design designated "C" with thermally conductive paste between the plates performed slightly better than the previous embodiment.

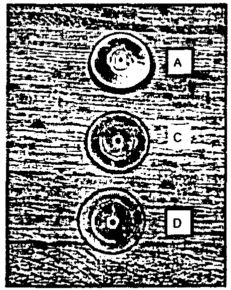

The goal was to obtain sufficient data on which type of plate performed better than the previous design. The experiments were performed using the following materials and methods.

Equipment used:
(i) DC variable power supply (KELVI ID 0024);
(ii) Flow meter (KELVI ID 0049);
(iii) Dual temperature sensor (KELVI ID 0016);
(iv) AC variable power supply (KELVI ID 0075); and
(v) Power meter (KELVI ID 0079).

Briefly, (i) Utility fluid channels were manufactured with various plate configurations. Then, (ii) the fluid channels were clamped to a thermal test fixture. Then, (ii) a heating pad was placed on top of the thermal test fixture. Then, (iii) a fixed volume of water was circulated through the fluid channel at a constant 2.0 LPM flow rate while measuring the temperature of the thermal test fixture. Then, (iv) the heating pad was tumed on at test time = 1 min. and kept constant at 450W for the duration of the test (6 min.).

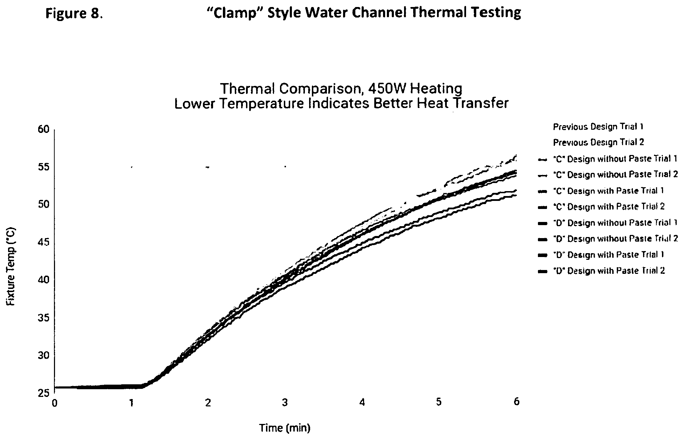

As is shown in **Figure 8****,** the heat transfer compared with each design is as follows. The "C" design without thermally conductive paste between the plates does not perform as well as the previous design and thus is not considered as a suitable alternative. However, the "C" design with a thermally conductive paste between the plates and the "D" design without thermally conductive paste between the plates perform equal to or better than the previous design. Finally, the "D" design with thermally conductive paste between the plates shows significant improvement over the previous design.

### Example 2: Simulated HEM Testing.

To further evaluate the results in the previous example, a simulated HEM test was performed using the following protocols.

Equipment used:
(i) DC variable power supply (KELVI ID 0036);
(ii) DC variable power supply (KELVI ID 0048);
(iii) Flow meter (KELVI ID 0049);
(iv) Dual temperature sensor (KELVI ID 0016);
(v) AC variable power supply (KELVI ID 0075); and
(vi) Power meter (KELVI ID 0079).

Briefly, (i) a Utility fluid channel with the previous embodiment plate design was clamped to a thermal test fixture with a TEC array using thermally conductive paste. Then, (ii) a radiator with fans (at constant 7V) were added in the water circulation loop. Then, (iii) a heating pad was placed on top of the thermal test fixture. Then, (iv) a fixed volume of water was circulated through the fluid channel at a constant 2.0 LPM flow rate. Then, (iv) the heating pad was turned on at test time = 1 min. and kept constant at 450W. Then, (v) TECs were turned on at test time = 90 sec. and kept at a constant 24V. Then, (vi) the temperature on the thermal test fixture was measured for the duration of the) 30 min. test. Then, (vii) the test was repeated using a Utility fluid channel with "C" design plates with thermally conductive paste between the plates. Finally, (viii) the previous two tests were repeated with the heating pad powered off for the duration of the test.

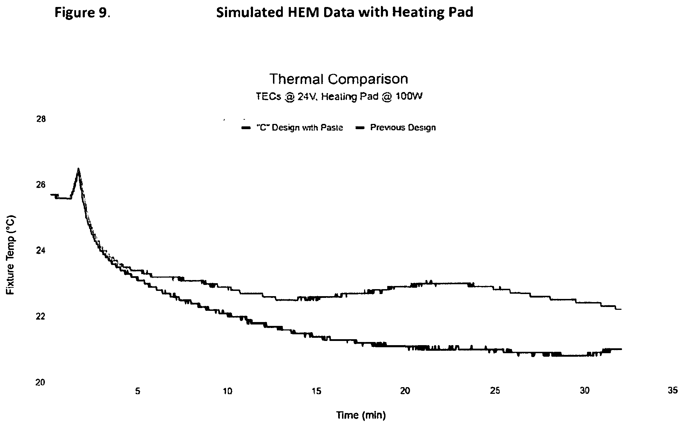

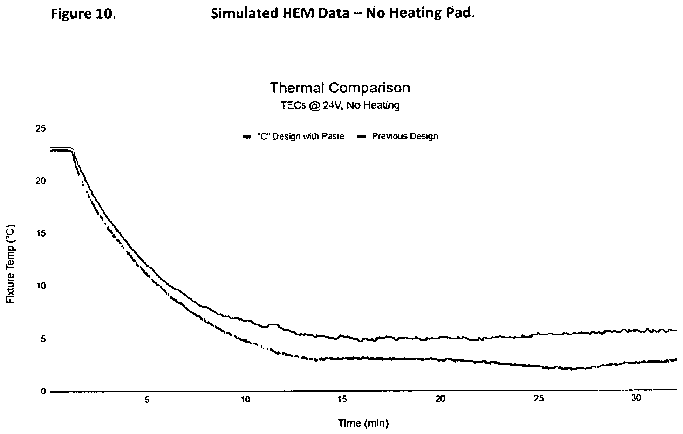

The results of the tests with heading pad showed that the "C" design with paste performed better than the previous design. **(****Figure 9****).** Furthermore, the same experiment without a heating pad showed that similar to the previous results, the "C" design with a thermally conductive paste performed better that the previous design. **(****Figure 10****).**

### Example 3: Differential Temperature Testing

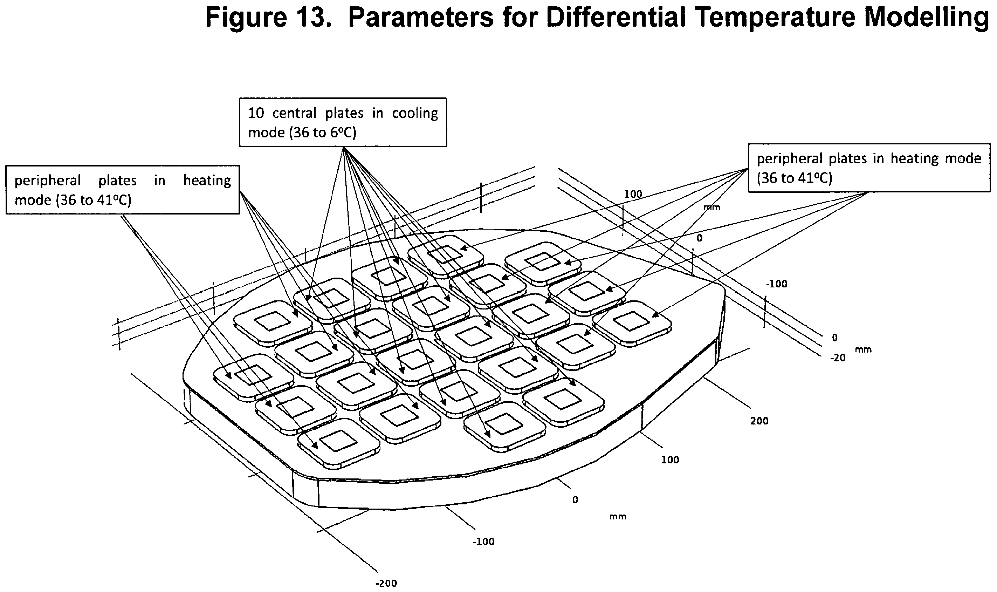

To further evaluate the ability to provide differential temperature by a plurality of TECs, a differential temperature model is developed. Briefly and for the purposes of this model, a back HEM is used with a geometry comprising twenty-four (24) skin contact plates, which consist of each plate having one (1) TEC located at the center (approx. 4.5 cm²). The area per contact plate is approximately 26.35 cm². The total skin contact area is approximately 598 cm². Further parameters of the model are assumed that the skin is approximately 1 mm thick, the muscle layer is approximately 25 mm thick, and the initial temperature of the study is 36° C. (See, **Figure 13****).**

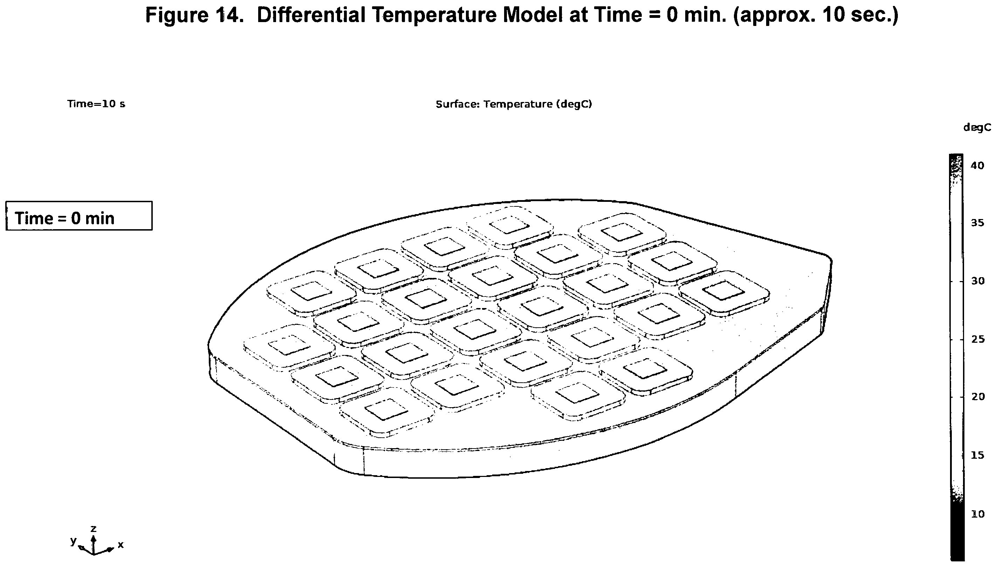

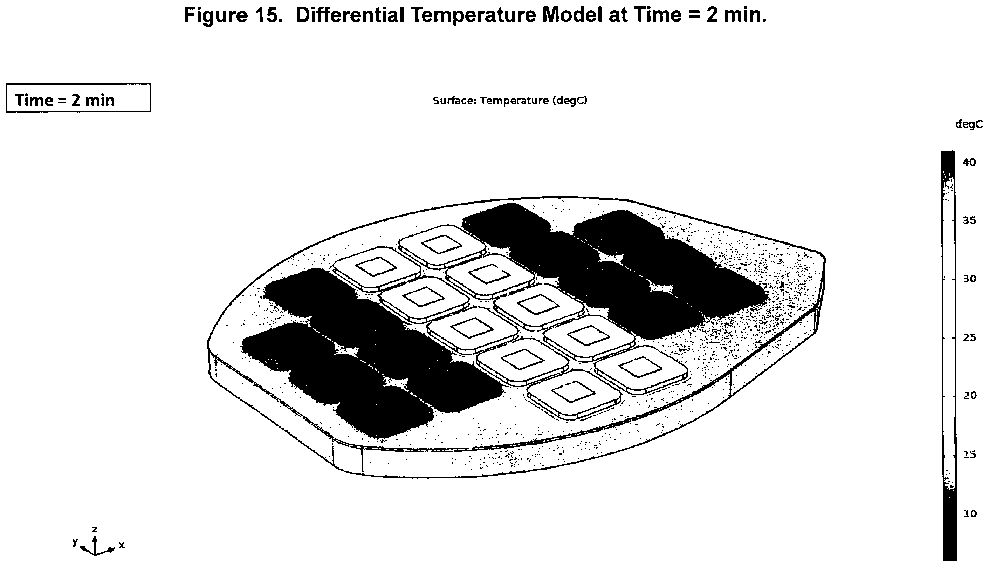

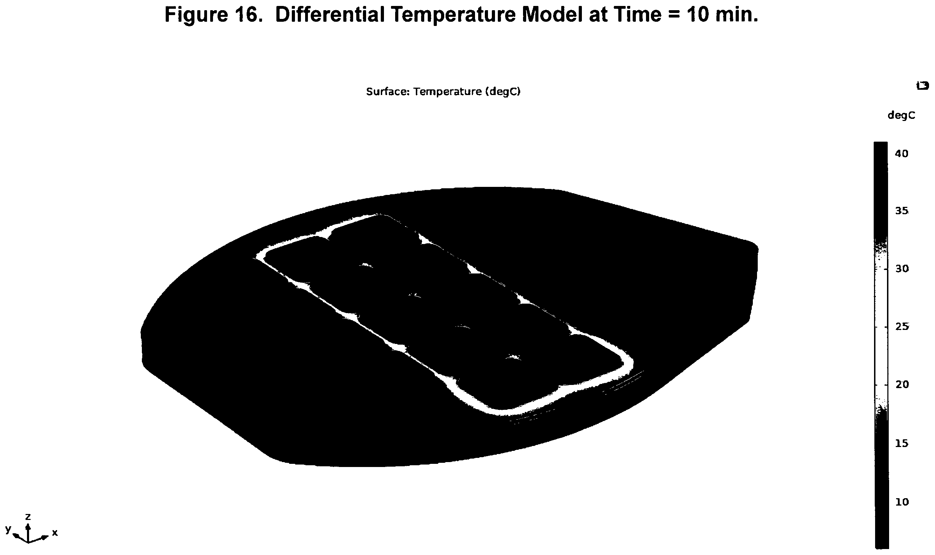

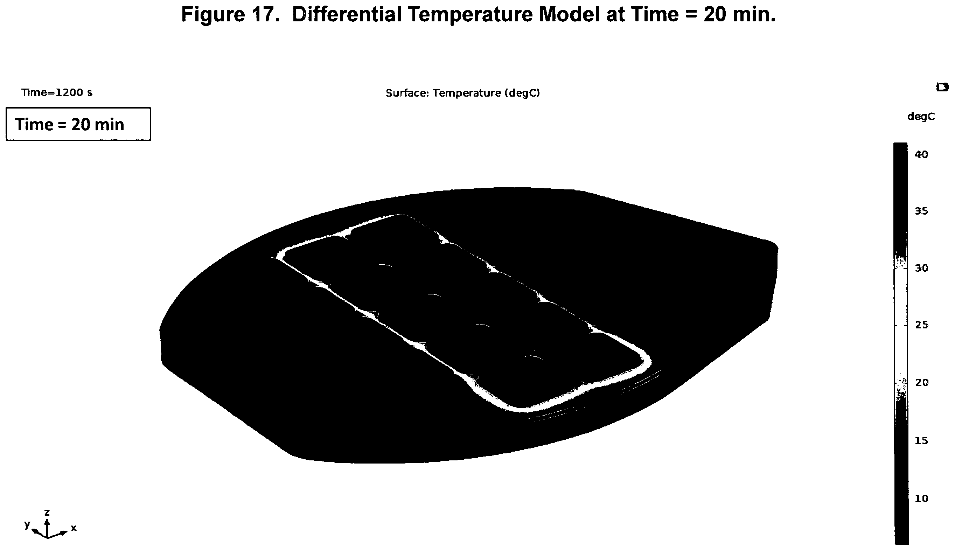

The results show that at time = 0 min., the surface temperature equals 36° C. **(****Figure 14****).** Further, at time = 2 min., the surface temperature of the middle plates has dropped, whereby the surface temperature of the side plates remains the same. **(****Figure 15****).** At time = 10 min., the surface temperature of the middle plates has continued to drop, while the outside plates have gone up in temperature. **(****Figure 16****).** Finally, at time = 20 min., the surface temperature of the middle plates has achieved a set (or pre-set) temperature drop to 6° C, while the surface temperature of the outside plates achieves a set (or pre-set) temperature of 41° C. **(****Figure 17****).**

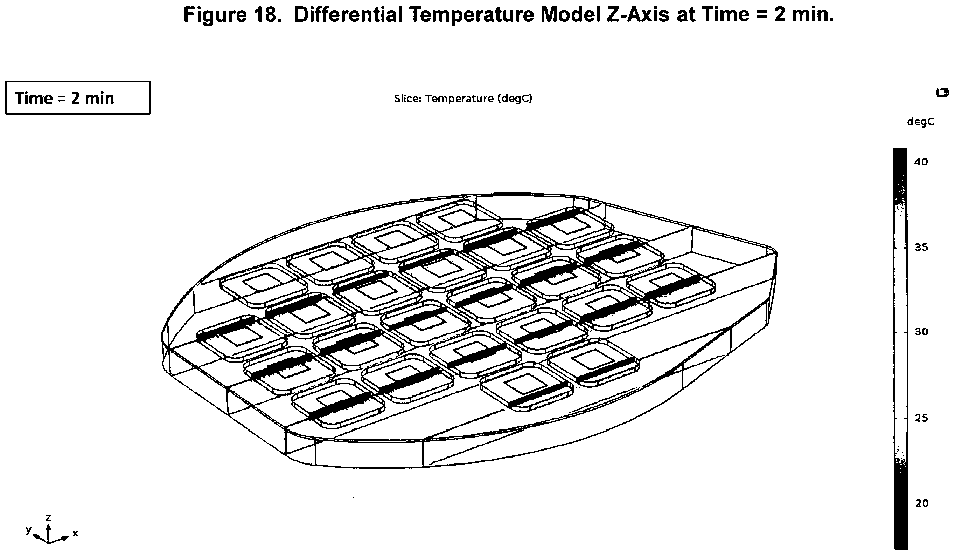

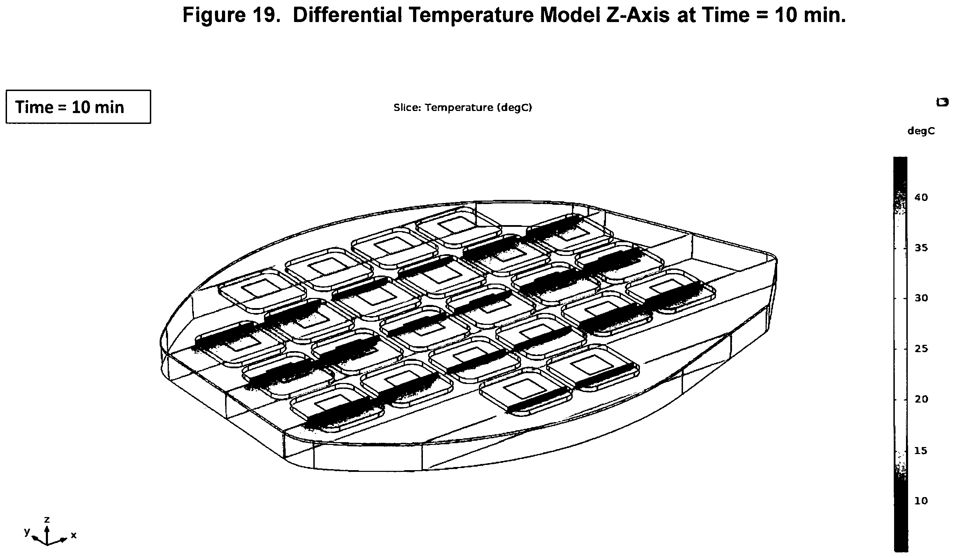

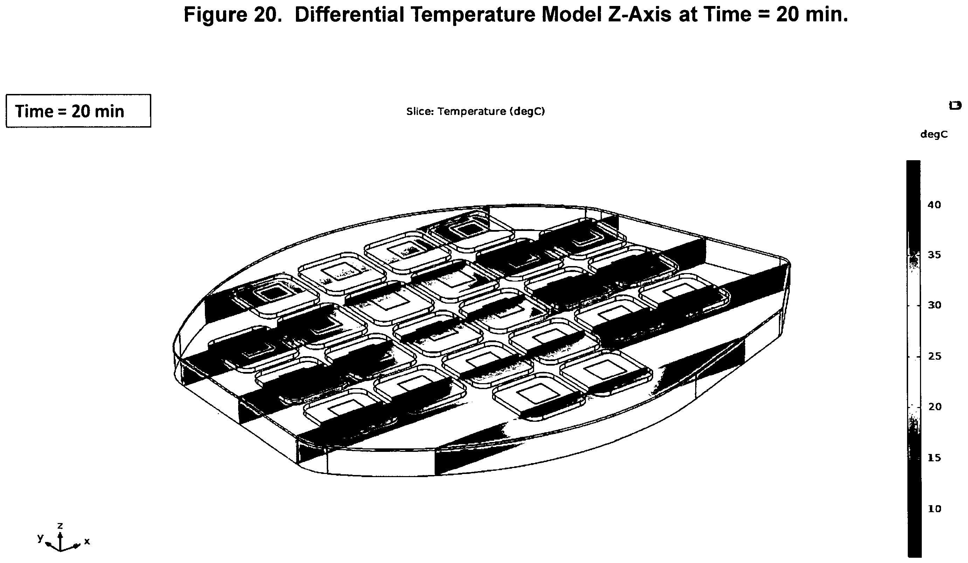

Additionally, **Figure 18****,** **Figure 19****,** and **Figure 20****,** show a slice pattern measuring the z-axis temperature,

The results of this model further demonstrate that the utilization of a differential temperature system within a traditional HEM, a HEM utilizing flexible TECs, or a fixed frame hand or foot station allows a user to target specific temperatures at portions of the body at a specific time. The principal advantages of this approach allow and end user or patient to access an ample spectrum of personalized thermal therapeutic modalities to various body parts using ergonomically designed devices, comprising not only periodic cooling and heating phases on a targeted area, but also on a plurality of proximal target areas.

One of skill in the art will appreciate and understand the unique advantages using the disclosed "differential" modalities in which a contact area may be in a "cooling" phase while the contours are in a "heating" phase.

The present disclosure opens novel and useful avenues of thermal therapy that allows for more effective recovery from injuries that the known standard of care of applying sequential application of heating and cooling phases (a.k.a. contrast therapy).

### Example 4: Fluid Channel Thermal Testing.

An additional array of experiments was performed to determine the optimization of the placement of a conductive thermal paste (See, **Example 1, "Clamp" Style Fluid Channel Thermal Testing)** within the fluid plate. Briefly, multiple back wraps comprising three (3) types were tested: (i) square water plates; (ii) "clamp" style (round) water plates with a thermally conductive paste at the interface between the first and second plate to ensure proper surface contact for heat transfer; and (iii) round water plates without a conductive paste:

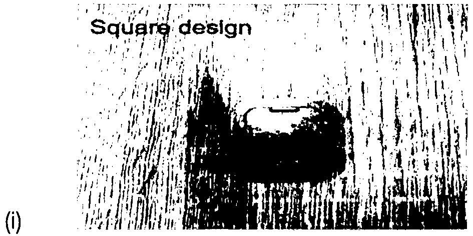

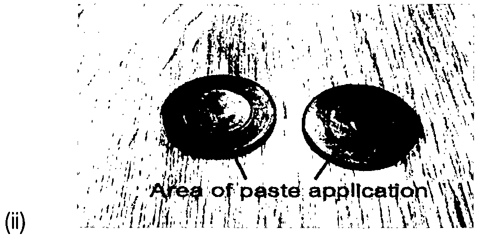

First, heating pads were placed on the top of the skin interface layer. To ensure consistent thermal contact the stack-up was clamped together. The water circulation was limited to 2.12 LPM with ball valve for consistency. Concurrently, an external ambient temperature sensor was used to maintain consistent ambient temperatures. The heating pad was turned to a power of 100W (as measured by both a current and voltage meter) which equates to 0.12 W/cm²on the back. After one (1) min., cooling began on the back wrap at a constant 18.1 V. The test was performed for thirty (30) minutes to allow for steady-state conditions to be reached. At thirty (30) minutes data was collected and parsed into CSV format with a Parlay data processer, allowing new collection of data on the individual tile level for periods > 30 seconds.

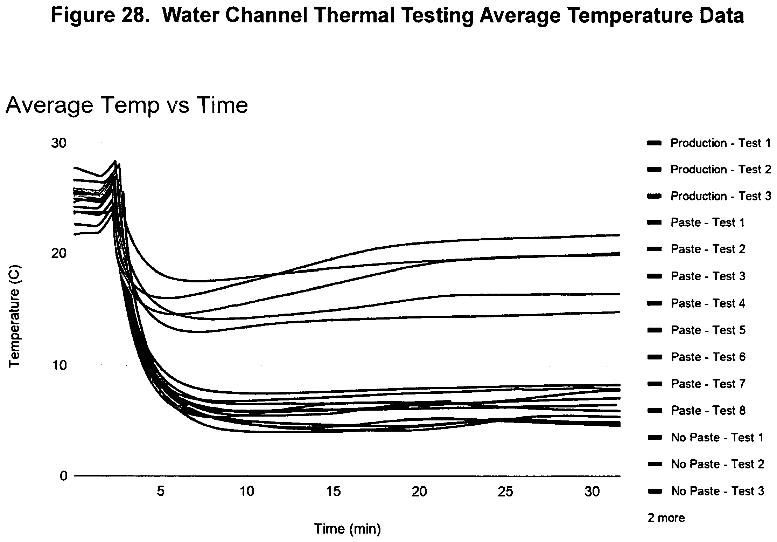

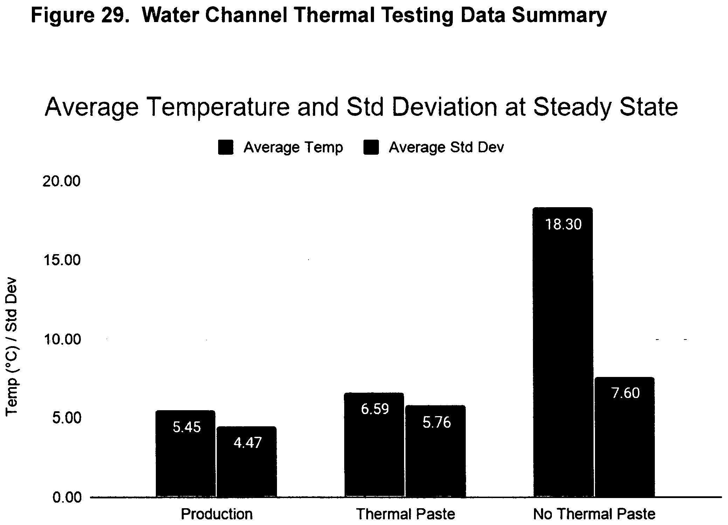

The results in **Figure 28** show that utilizing a round plate with a conductive thermal paste works significantly better that a round plate without thermal paste. In addition, the square plates performed within an acceptable range as the round plate with thermal paste. However, upon inspection of the round plates, it was determined that the embedding temperature was too low. Thus, in the round plates with paste, it was shown that the conductive paste was effectively bridging the gap between the plates to allow for sufficient heat transfer despite suboptimal embedding on the round plates. See, **Figure 29****.** Accordingly, the temperature of the embedding tool must be increased during production.

### Example 5: Evaluation of Bond Strength (Embedding) in Fluid Channels.

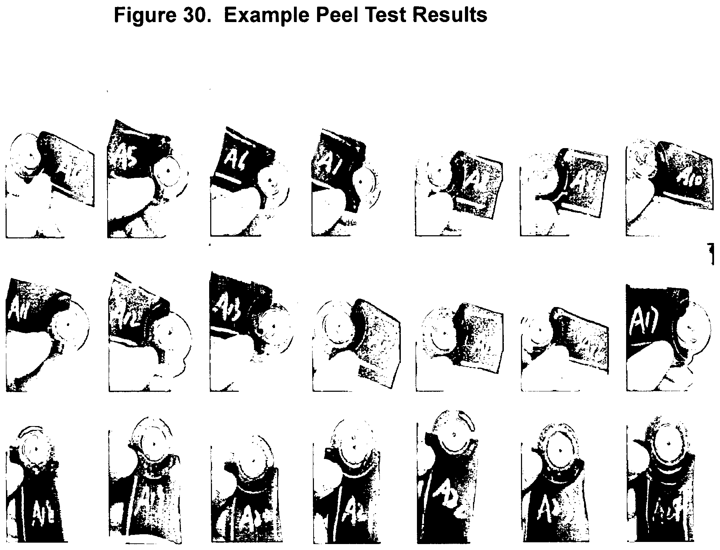

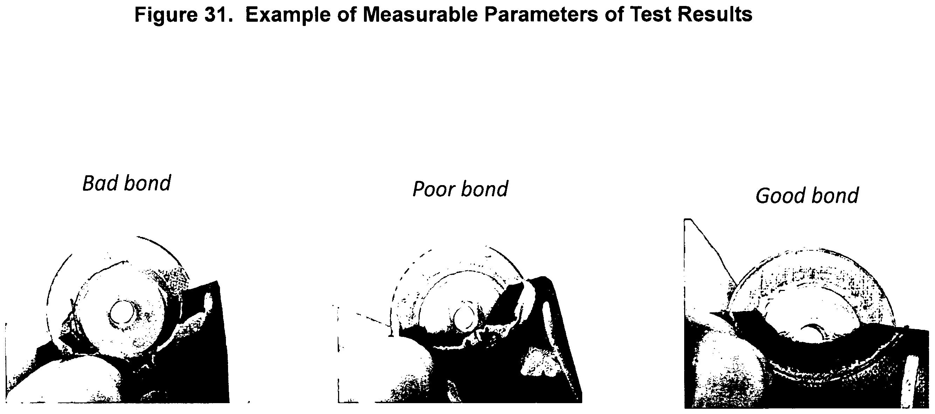

In another set of experiments bond strength (embedding) between the TPU and metal fluid plates was evaluated via a "peel test" in which a sheet of fabric backed TPU is heat pressed (a.k.a. embedded) on a set of fluid plates and then removed by force leaving a material pattern visible on the metal plate. Upon viewing material pattern when the bond strength is high, the TPU will separate from its fabric backing and remain on the metal plate. However, when the bond strength is low, the TPU will separate from the metal and stay with the fabric. An example of peel test results as well as the measurable parameters is set forth in **Figure 30** and **Figure 31****.**

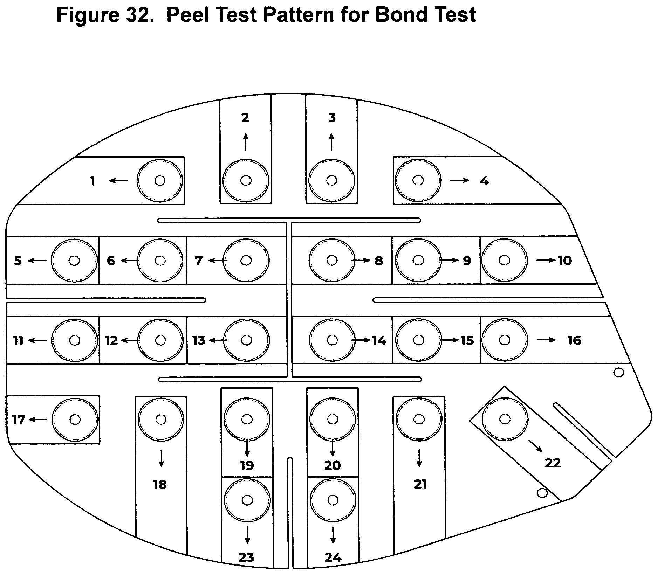

The bond strength test was performed using the following protocols. First, A full embed is performed following the pattern of the water channel. The embedded layer is then numbered and cut into strips so each plate can be peeled individually. See, **Figure 32****.** For the square plate(s) only one (1) sheet is embedded and peeled. For the round "clamp" style plate as set forth in this disclosure only one plate is coated with the adhesive primer that allows for embedding. This allows the unbonded plate to be removed so the bonded side can be examined. It is noted that if both sides are bonded, it is not possible to perform the peel test without damaging the bonding surface. The plate is held in place and the TPU strip is peeled away to reveal the bonding surface. Information can be determined by the appearance of the peeled bonding surface via physical inspection. (See, **Figure 30** **&** **Figure 31****).** A consistent texture and lack of air bubbles in the TPU indicates that the tool temperature was in the correct range. Note, too low of a temperature and the TPU will not bond to the metal, too high of a temperature and the TPU will boil and leave air gaps which can cause water leaks in a finished channel.

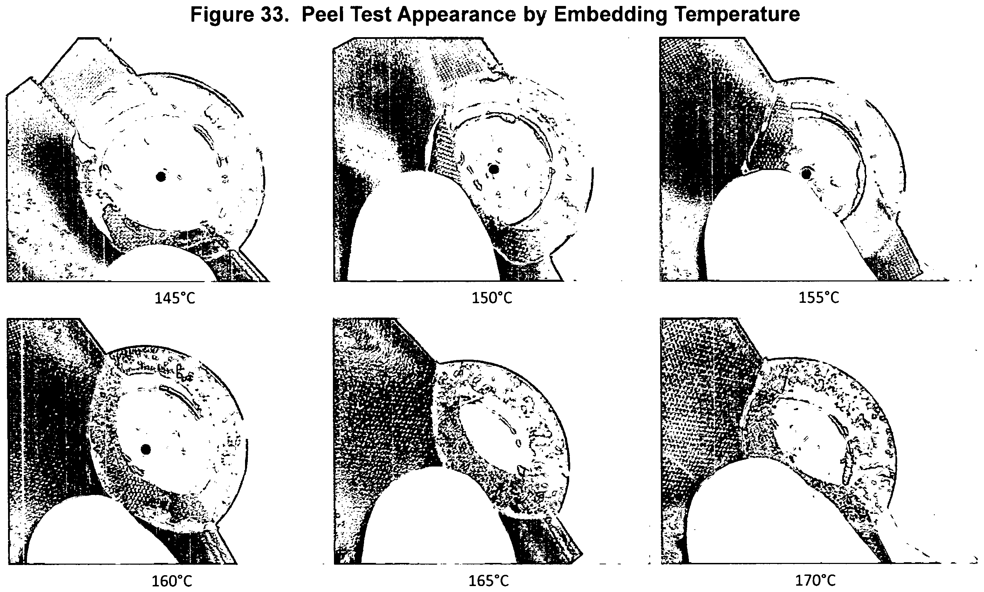

The results in **Figure 33** shows a peel test appearance of a round plate by embedding temperature. The results show the acceptable range of embedding temperature is approximately 150-160°C.

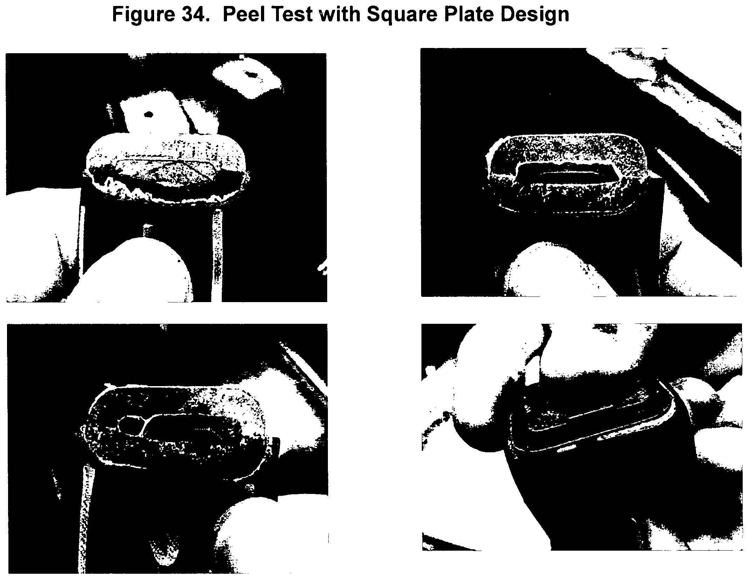

The results in **Figure 34** shows the square plates that feature TPU bonding on both sides of a single metal plate. Direct compression of the TPU during embedding can displace too much TPU and lead to a weaker bond. The test also reveals that the direction of force applied to the TPU can influence the separation pattern. Additionally, there is no mechanical protection or covering of the bonded area which can allow water ingress through the exposed edges of the TPU material where the perforations are cut.

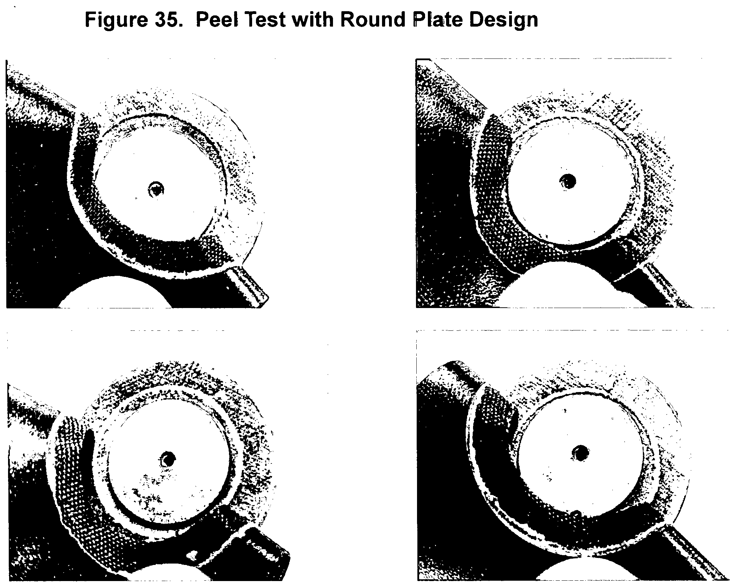

The results in **Figure 35** shows the round plates that feature a single sheet of TPU bonded to metal on both sides. The redundant metal bond provides physical protection of the bond area and makes a single continuous leak less likely. The fixed gap dimension between the top and bottom plate prevents excessive displacement of TPU during embedding which results in a stronger bond. Force applied from any direction produces consistent stress on the circular shape. Additionally, the cut edge of the TPU is concealed from water, preventing water ingress through the fabric.

Taken together these results show that the round plate design (i) is more likely to form a stronger bond, (ii) evenly distributes stress applied to the fluid channel which decreases probability of failures caused by concentrated stress, and (iii) conceals the cut edge of the TPU from direct exposure to water thereby preventing ingress through the material which can lead to material degradation or fluid leaks.

Although the description herein contains many details, these should not be construed as limiting the scope of the disclosure but as merely providing illustrations of some of the presently preferred embodiments. Therefore, it will be appreciated that the scope of the disclosure fully encompasses other embodiments provided they fall under the scope of the appended claims.

## Claims

1. An apparatus having a fluid channel, wherein the fluid channel comprises:
a first layer (200) comprising cut-outs (210), a second layer (240), a first plate (220) and a second plate (230) embedded between the first and second layer and directly in contact with fluid flowing in the fluid channel, wherein each of the first layer and the second layer is made from a flexible material;
wherein the first and second plates are clamped by mechanical fasteners at the point of the cut outs, to create a seal against the first layer, and wherein the first plate and the second plate are interfaced with a thermally conductive paste.

2. The apparatus of claim 1, wherein the second layer (240) is attached onto the first layer to create a continuous fluid path (520) about the second plate.

3. The apparatus of claim 1 or claim 2, whereby the first layer is thermoplastic polyurethane (TPU).

4. The apparatus of any of claims 1 to 3, wherein the cut-outs are shaped in a uniform grid.

5. The apparatus of any of claims 1 to 4, further comprising a stand-off (250), wherein the stand-off is attached to material on a side opposite to an elevated platform (510) of the first plate for maintaining fluid flow and prevent channel collapse.

6. An article of manufacture comprising an apparatus according to any of claims 1 to 5.

7. A heat exchange module (HEM) comprising the apparatus of any one of claims 1 to 5.

8. A heat exchange module (HEM) as claimed in claim 7, further comprising:
a fixed frame therapy station (700) comprising aluminium, wherein the fixed frame is molded in the shape of a human hand or a human foot; and
a controller.

## Patentansprüche

1. Einrichtung, die einen Fluidkanal aufweist, wobei der Fluidkanal Folgendes umfasst:
eine erste Schicht (200), die Aussparungen (210) umfasst, eine zweite Schicht (240), eine erste Platte (220) und eine zweite Platte (230), die zwischen der ersten und der zweiten Schicht eingebettet sind und in direktem Kontakt mit einem im Fluidkanal fließenden Fluid stehen, wobei sowohl die erste Schicht als auch die zweite Schicht aus einem flexiblen Material bestehen;
wobei die erste und die zweite Platte am Punkt der Aussparungen mittels mechanischer Verbindungselemente zusammengeklemmt werden, um eine Abdichtung gegen die erste Schicht zu erzeugen, und wobei die erste Platte und die zweite Platte mit einer wärmeleitenden Paste verbunden sind.

2. Einrichtung nach Anspruch 1, wobei die zweite Schicht (240) an der ersten Schicht befestigt ist, um einen durchgehenden Fluidpfad (520) um die zweite Platte herum zu erzeugen.

3. Einrichtung nach Anspruch 1 oder Anspruch 2, wobei die erste Schicht thermoplastisches Polyurethan (TPU) ist.

4. Einrichtung nach einem der Ansprüche 1 bis 3, wobei die Aussparungen in einem gleichmäßigen Raster geformt sind.

5. Einrichtung nach einem der Ansprüche 1 bis 4, ferner umfassend einen Abstandshalter (250), wobei der Abstandshalter an einem Material auf einer Seite gegenüberliegend einer erhöhten Plattform (510) der ersten Platte befestigt ist, um einen Fluidstrom aufrechtzuerhalten und ein Zusammenbrechen des Kanals zu verhindern.

6. Fertigungsartikel, umfassend eine Einrichtung nach einem der Ansprüche 1 bis 5.

7. Wärmetauschermodul (HEM), umfassend die Einrichtung nach einem der Ansprüche 1 bis 5.

8. Wärmetauschermodul (HEM) nach Anspruch 7, ferner umfassend:
eine Therapiestation (700) mit festem Rahmen, die Aluminium umfasst, wobei der feste Rahmen in der Form einer menschlichen Hand oder eines menschlichen Fußes geformt ist; und
eine Steuereinheit.

## Revendications

1. Appareil présentant un canal de fluide, dans lequel ledit canal de fluide comprend :
une première couche (200) comprenant des découpes (210), une seconde couche (240), une première plaque (220) et une seconde plaque (230) intégrées entre la première et la seconde couche et directement en contact avec le fluide circulant dans le canal de fluide, dans lequel chacune de la première et de la seconde couche est constituée d'un matériau flexible ;
dans lequel les première et seconde plaques sont serrées par des fixations mécaniques au niveau des découpes, afin de créer un joint contre la première couche, et dans lequel la première plaque et la seconde plaque sont reliées par une pâte thermoconductrice.

2. Appareil selon la revendication 1, dans lequel la seconde couche (240) est fixée sur la première couche pour créer un chemin de fluide continu (520) autour de la seconde plaque.

3. Appareil selon la revendication 1 ou la revendication 2, selon lequel la première couche est en polyuréthane thermoplastique (TPU).

4. Appareil selon l'une quelconque des revendications 1 à 3, dans lequel les découpes sont formées selon une grille uniforme.

5. Appareil selon l'une quelconque des revendications 1 à 4, comprenant en outre un entretoise (250), dans lequel l'entretoise est fixée à un matériau sur un côté opposé à une plateforme surélevée (510) de la première plaque pour maintenir le flux de fluide et empêcher l'effondrement du canal.

6. Article de fabrication comprenant un appareil selon l'une quelconque des revendications 1 à 5.

7. Module d'échange de chaleur (HEM) comprenant l'appareil selon l'une quelconque des revendications 1 à 5.

8. Module d'échange thermique (HEM) selon la revendication 7, comprenant en outre :
une station de thérapie à cadre fixe (700) comprenant de l'aluminium, dans lequel le cadre fixe est moulé en forme de main ou de pied humain ; et
un dispositif de commande.
